# EUROPÄISCHE PATENTSCHRIFT

(11) **EP 0 421 271 B1**
(45) Veröffentlichungstag und Bekanntmachung des Hinweises auf die Patenterteilung: **08.12.1993**
(21) Anmeldenummer: 90118534.8
(22) Anmeldetag: 27.09.1990
(51) Int. Cl.: C07C 69/716, C07C 69/738, C07C 67/343, C07C 67/317

(54) **3-substituierte 2-Hydroxy-3-formylpropionsäureester, Verfahren zu deren Herstellung sowie deren Verwendung zur Herstellung von 3-substituierten 3-Formyl-acrylsäurereestern**
3-Substituted-2-hydroxy-3-formyle propionic acid esters, process for their preparation and their use in the preparation of 3-substituted-3-formyle acrylic acid esters
Esters de l'acide 2-hydroxy-3-formyle propionique substitué en 3, leur procédé de préparation et leur utilisation pour la préparation d'esters d'acide 3-formyle acrylique

(30) Priorität: 06.10.1989 DE 3933334
(43) Veröffentlichungstag der Anmeldung: 10.04.1991
(73) Patentinhaber: BASF Aktiengesellschaft, 67063 Ludwigshafen (DE)
(72) Erfinder: Merger, Franz, Dr., W-6710 Frankenthal (DE); Frank, Juergen, Dr., W-6703 Limburgerhof (DE)

(56) Entgegenhaltungen:
- DE-A- 2 542 366
- DE-A- 3 617 409
- DE-B- 1 008 729
- JOURNAL OF THE AMERICAN CHEMICAL SOCIETY. Bd. 103, Nr. 9, 6. Mai 1981, GASTON, PA US Seiten 2409 - 2410; Y. OHFUNE: 'Total synthesis of 2 -Deoxymugineic acid'

## Beschreibung

Die Erfindung betrifft 3-substituierte 2-Hydroxy-3-formyl-propionsäureester der allgemeinen Formel I 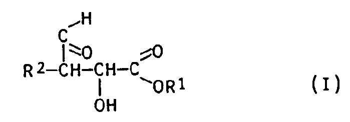
in der R¹ für einen Alkylrest mit 1 bis 4 C-Atomen steht
und R² für einen geradkettigen oder verzweigten Alkylrest mit 1 bis 10 C-Atomen steht, ein Verfahren zu deren Herstellung sowie deren Verwendung zur Herstellung von 3-substituierten 3-Formyl-acrylsäureestern.

3-substituierte 3-Formyl-acrylsäureester sind gesuchte Vorprodukte für die Herstellung von pharmakologisch wirksamen Verbindungen.

Im einfachsten Fall, wenn der Substituent in der 3-Position eine Methylgruppe ist, handelt es sich beispielsweise um den für die Produktion von Vitamin-A-Säure gesuchten 3-Formyl-crotonsäureester.

Neben einer Reihe von Laboratoriumsmethoden, die durch Oxidation einer der beiden Methylgruppen von Estern der Seneciosäure (3,3-Dimethyl-acrylsäure) zu Estern der Formylcrotonsäure führen (vgl. J. Am. Chem. Soc. 82, (1960) 2286 - 88; Tetrahedron Letters 1976, 3985 - 86; Synthesis 1983, 297 -300), sind auch schon Verfahren bekannt, welche zur technischen Produktion der begehrten Verbindungen benutzt werden können.

Jedoch sind selbst die besten Verfahren noch heute mit schwerwiegenden Nachteilen verknüpft. Beispielsweise sind die Umsetzungen zwischen Acetalen des Methylglyoxals und Phosphonoessigsäureestern nach Wittig-Horner dadurch nachteilig, daß die Ausgangsmaterialien in mehrstufigen Synthesen hergestellt und hierfür toxische oder aggressive Substanzen gehandhabt werden müssen. So wird z.B. Methylglyoxaldimethylacetal über die Nitrosierung von Aceton produziert (vgl. Pure Appl. Chem. 43, (1975) 540). Zur Darstellung der zweiten erforderlichen Komponente, dem Phosphonoessigester, muß Trialkylphosphit mit dem stark tränenreizenden Bromessigester zur Reaktion gebracht werden. Die als Nebenprodukte entstehenden Alkylbromide kommen mehr und mehr unter Verdacht, krebserzeugendes Potential zu besitzen. Insgesamt ist die Wittig-Horner-Synthese durch diese Komplikationen schwierig zu kontrollieren, aufwendig und damit wenig wirtschaftlich.

Art und Weise der Durchführung der Wittig-Horner-Reaktion sind literaturbekannt (vgl. z.B. J. Chem. Soc., Perkin Trans. I, 1987, 1743 - 48).

Weiterhin ist aus DE 1 008 729 bekannt, daß alpha-Hydroxy-alpha-alkoxyessigsäureester mit Propionaldehyd unter Katalyse mit Dialkylaminen zu 3-Formyl-crotonsäureestern kondensiert werden können. Gemäß diesem Verfahren werden die Komponenten zur Durchführung der Reaktion vorgelegt und durch Zusatz des Katalysators die exotherme Reaktion in Gang gebracht.

Diese Arbeitsweise ist aus Sicherheitsgründen in technischem Maßstab nicht durchführbar (s. Vergleichsbeispiele 1 bis 3). Die erreichbaren Ausbeuten sind darüber hinaus nicht voll befriedigend.

Außerdem ist aus DE-OS 25 42 366 (vgl. Seite 16, Zeilen 9-10) die Herstellung von 3-Formyl-crotonsäurebutylester durch Versetzen eines Gemisches aus Glyoxalsäurebutylester und Propionaldehyd mit Di-n-butylamin unter Verhinderung eines Temperaturanstiegs über 106°C und anschließendes 2-stündiges Rühren des Reaktionsgemisches bei 106 bis 110°C bekannt.

Auch diese Arbeitsweise ist aus Sicherheitsgründen in technischem Maßstab nicht durchführbar und bezüglich der Ausbeuten nicht befriedigend.

In einem neueren Artikel in J. Org. Chem. 52 (1987), Seiten 4788-90, wird ausführlich dargelegt, welche schwerwiegenden Probleme in verschiedenen Arbeitskreisen auftraten, als man versuchte, Glyoxylsäure mit Carbonylverbindungen umzusetzen. Besondere Schwierigkeiten traten auf bei Versuchen, aliphatische Aldehyde mit Glyoxylsäure umzusetzen (vgl. loc. cit. Seite 4788). Beispielsweise wurden bei der Umsetzung von aliphatischen Aldehyden mit Glyoxylsäure in Gegenwart von Morpholin im wesentlichen nur Butenolide gebildet. Als Ausweg aus diesem Dilemma wird in loc. cit. angegeben, daß man 3-Formyl-3-alkyl-acrylsäureester recht gut erhalten kann, wenn man Enamine der Aldehyde mit Glyoxylsäureestern bzw. Glyoxylsäureester-halbacetalen umsetzt und anschließend die erhaltenen Addukte hydrolysiert. Nachteilig an diesem Verfahren ist, daß nicht die günstig verfügbaren freien Alkanale eingesetzt werden können, sondern deren Enamine verwendet werden müssen. Speziell die Enamine niederer Alkanale sind aber technisch nicht günstig zugänglich. Des weiteren muß die Hydrolyse unter genau einzuhaltenden Bedingungen erfolgen (s. loc.-cit. S. 4789, rechte Spalte, Zeilen 18-20). Außerdem muß das als Hilfsreagenz verwendete Amin in stöchiometrischer Menge eingesetzt werden und liegt nach der Hydrolyse in mineralsaurer Lösung vor. Aus dieser ist es nur auf aufwendige Weise durch Neutralisation und Extraktion bzw. Destillation wiederzugewinnen.

Es war daher die Aufgabe der Erfindung, ein günstigeres Verfahren zur Herstellung von 3-substituierten 3-Formyl-acrylsäureestern aus Glyoxylsäureestern und Alkanalen zu entwickeln, das es erlaubt von freien Alkanalen auszugehen, ohne daß die Nachteile des Standes der Technik auftreten.

Es wurde nun überraschenderweise gefunden, daß man die gewünschten 3-substituierten 3-Formylacrylsäureester durch Umsetzen von Glyoxylsäureestern mit freien Alkanalen in Gegenwart von Gemischen bzw. Salzen von sekundären Aminen und Carbonsäuren oder aber in Gegenwart von Verbindungen, die eine sekundäre Aminogruppe und eine Carboxylgruppe im gleichen Molekül enthalten, mit recht guten Ausbeuten erhält, wenn man die Glyoxylsäureester mit den Alkanalen zunächst unter definierten milden Reaktionsbedingungen in die neuen 3-substituierten 2-Hydroxy-3-formylpropionsäureester der Formel I überführt und aus diesen durch Behandeln mit einem wasserabspaltenden Mittel, vorzugsweise durch Behandeln mit Acetanhydrid, die gewünschten 3-substituierten 3-Formyl-acrylsäureester herstellt.

Man führt also die erfindungsgemäße Reaktion zwischen Glyoxylsäureester und Alkanal in zwei Stufen durch. Zur Herstellung der neuen 2-Hydroxy-3-formyl-propionsäureester der Formel I dosiert man die Reaktionskomponenten gleichzeitig, vorzugsweise in Form einer Mischung, insbesondere einer äquimolare Mischung aus Glyoxylsäureester und Alkanal langsam zu dem vorgelegten speziellen Katalysatorsystem zu oder aber dosiert das Alkanal langsam zu einem Gemisch aus dem oben beschriebenen Katalysatorsystem und dem Glyoxylsäureester hinzu. Auf diese Weise kann man die Wärmeentwicklung sehr gut kontrollieren. Glyoxylsäureester und Alkanal vereinigen sich in diesem ersten Reaktionsschritt zu den neuen 3-monosubstituierten 2-Hydroxy-3-formylpropionsäureestern der Formel I.

Die erfindungsgemäße Arbeitsweise überschneidet sich in den Maßnahmen mit dem Grenzbereich der Maßnahmen, die in DE 36 17 409 zur Kondensation von Glyoxalmonoacetalen mit Alkanalen gelehrt werden. Während die Kondensation dort besonders bevorzugt in Gegenwart von 20 bis 100 Mol.-% Katalysator bezüglich Ausgangsmaterial mit sehr gutem Erfolg durchgeführt wird, gelingt die Kondensation von Glyoxylsäuremethylester mit Propanal unter diesen Bedingungen unter hohem Aufwand nur mit mäßiger Ausbeute und liefert ein verfärbtes und nicht lagerstabiles Produkt (s. Beispiel Nr. 4).

In Gegenwart sehr geringer Mengen von Katalysatoren bestehend aus Carbonsäure und sek. Amin bilden sich aus Glyoxylsäureester bzw. Glyoxylsäureesterhalbacetal mit Alkanalen dagegen umso überraschender nicht die erwarteten 3-Formyl-3-alkylacrylsäureester, sondern die neuen 2-Hydroxy-3-formyl-3-alkylpropionsäureester.

Gegenstand der Erfindung ist daher auch ein Verfahren zur Herstellung der neuen 3-substituierten 2-Hydroxy-3-formyl-propionsäureestern der allgemeinen Formel I 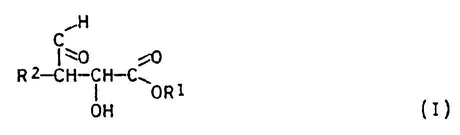
in der
R¹ für einen Alkylrest mit 1 bis 4 C-Atomen steht und
R² für einen geradkettigen oder verzweigten Alkylrest mit 1 bis
10 C-Atomen steht, das dadurch gekennzeichnet ist, daß man
ein Alkanal der allgemeinen Formel II 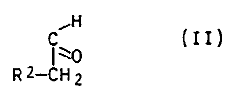
und einen Glyoxylsäurealkylester der allgemeinen Formel III 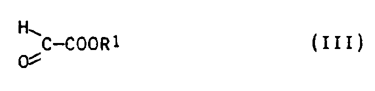
in denen R¹ und R² die oben angegebene Bedeutung haben, entweder gleichzeitig zu einem Katalysatorsystem bestehend aus einem Salz eines sekundären Amins mit einer Carbonsäure oder einem etwa äquimolekularen Gemisch aus einem sekundären Amin und einer Carbonsäure oder einer Verbindung, die eine sekundäre Aminogruppe und eine Carboxylgruppe im gleichen Molekül enthält, so zudosiert, daß die Temperatur 90°C, vorzugsweise 80°C, nicht übersteigt oder aber das Alkanal der Formel II zu einem Gemisch aus dem oben beschriebenen Katalysatorsystem und dem Glyoxylsäureester der Formel III so zudosiert, daß die Temperatur 90°C, vorzugsweise 80°C, nicht übersteigt, wobei das Katalysatorsystem in Mengen von 0,01 bis 10 Mol-%, vorzugsweise 0,1 bis 8 Mol-%, insbesondere 0,5 bis 5 Mol-%, verwendet wird.

Als Alkanale der Formel II können demnach Propionaldehyd, Butyraldehyd, Valeraldehyd, Isovaleraldehyd, isomere Hexanale, Heptanale, Octanale, Nonanale, Decanale, Undecanale und Dodecanale eingesetzt werden.

Besondere Bedeutung hat das erfindungsgemäße Verfahren für die Umsetzung von Glyoxylsäurealkylestern mit Alkanalen der Formel II, in der R² für einen Alkylrest mit 1 bis 5 C-Atomen steht, insbesondere für die Umsetzung mit Propionaldehyd.

Die Glyoxylsäurealkylester der Formel III können durch Oxydehydrieren der entsprechenden Glykolsäureester hergestellt werden. Die im Handel erhältlichen Produkte liegen in teilweise oligomerisierter Form vor, können aber durch Einwirkung von Spuren von Säuren wieder zurückgespalten werden. Von besonderer Bedeutung ist das erfindungsgemäße Verfahren für die Umsetzung des Glyoxylsäuremethylesters.

Als Katalysatoren für den ersten Reaktionsschritt eignen sich Salze bzw. 1/1-Gemische aus einem sekundären Amin und einer Carbonsäure. Geeignete Amine sind aliphatische Amine, wie Dimethylamin, Diethylamin, Dipropylamin, Dibutylamin, Methylethylamin, Methylpropylamin, Methylbutylamin, Methylcyclohexylamin sowie substituierte Amine wie Methylethanolamin und Diethanolamin oder cyclische Amine wie Pyrrolidin, Piperidin oder Morpholin.

Als Carbonsäuren eignen sich z. B. Essigsäure, Propionsäure, Buttersäure, Valeriansäuren, Hexansäuren und 2-Ethyl-hexansäure, substituierte Carbonsäuren, wie Methoxyessigsäure oder Butoxyessigsäure.

Besonders wirksam sind solche Katalysatoren, die die Amin- und Säurefunktion im gleichen Molekül vereinigen, wie Piperidin-2-carbonsäure, Pyrrolidin-2-carbonsäure (Prolin) und insbesondere N-Methyl-aminoessigsäure (Sarkosin).

Die Katalysatoren verwendet man üblicherweise in Mengen von 0,01 - 10 Mol-% bezüglich des eingesetzten Glyoxylesters, bevorzugt sind 0,1 - 8 Mol-% und insbesondere 0,5 - 5 Mol-%.

Im allgemeinen führt man die Reaktion bei Temperaturen zwischen 20 und 90°C, vorzugsweise zwischen 30 und 80°C durch.

Die erfindungsgemäßen 2-Hydroxy-3-formyl-propionsäureester der Formel I kann man zur Herstellung der begehrten 3-substituierten 3-Formyl-acrylsäureester der allgemeinen Formel IV 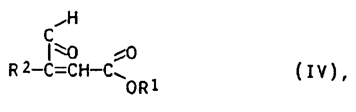
in der R¹ und R² die oben angegebene Bedeutung haben, mit einem wasserabspaltenden Mittel behandeln.

Die Abspaltung von Wasser aus den 2-Hydroxy-3-formyl-propionsäureestern der Formel I kann nach deren Isolierung vorgenommen werden. Mit besonderem Vorteil geht man jedoch direkt von dem erfindungsgemäß erhaltenen Reaktionsgemisch aus.

Hierbei kann man die Wasserabspaltung durch Behandlung der erfindungsgemäßen 2-Hydroxy-3-formyl-3-alkylpropionsäureester mit katalytischen Mengen einer Säure oder - vorzugsweise - durch Behandlung derselben mit Essigsäureanhydrid durchführen.

Obwohl das Erhitzen mit Essigsäureanhydrid zwecks Wasserabspaltung nur zur Umsetzung mit den leichter dehydratisierbaren 3-Hydroxycarbonsäurederivaten empfohlen wird (vgl. Houben-Weyl, Methoden der Organischen Chemie, Bd. 5/1b, S. 79, Georg Thieme Verlag Stuttgart, 1972), gelingt die Dehydratisierung der 2-Hydroxy3-formyl-3-alkylpropionsäureester zu 3-Formyl-3-alkylacrylsäureestern überraschenderweise besonders einfach durch Erhitzen mit Essigsäureanhydrid.

Man arbeitet so, daß man zu dem erfindungsgemäß erhaltenen Reaktionsgemisch Acetanhydrid in Mengen von 1 bis 5 Mol/Mol und ggf. geringe Mengen eines Katalysators, wie Natriumacetat oder 4-Dimethylamino-pyridin zufügt, anschließend noch 1 bis 12, vorzugsweise 2 bis 6 Stunden unter Rückfluß zum Sieden erhitzt, die gebildete Essigsäure abdestilliert und den Rückstand unter vermindertem Druck fraktioniert destilliert.

Auf diese Weise ergibt sich ein Gesamtverfahren, bei dem man die als Vorprodukte für Vitamin-A-Säure sehr begehrten 3-Formyl-crotonsäurealkylester der allgemeinen Formel IVa 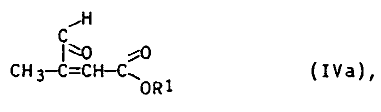
in der
R¹ für einen Alkylrest mit 1 bis 4 C-Atomen steht, in hohen Ausbeuten bis über 80 % erhalten kann und das dadurch gekennzeichnet ist, daß man
A. Propionaldehyd und einen Glyoxylsäurealkylester der allgemeinen Formel III 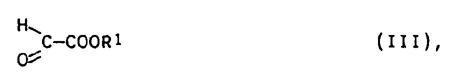 in der
   R¹ die oben angegebene Bedeutung hat,
   entweder gleichzeitig zu einem Katalysatorsystem bestehend aus einem Salz eines sekundären Amins mit einer Carbonsäure oder einem etwa äquimolaren Gemisch aus einem sekundären Amin und einer Carbonsäure oder einer Verbindung, die die gleiche Anzahl von sekundären Aminogruppen und Carboxylgruppen im gleichen Molekül enthält, so zudosiert, daß die Temperatur 90°C, vorzugsweise 80°C, nicht übersteigt, oder das Alkanal zu einem Gemisch aus dem oben beschriebenen Katalysatorsystem und dem Glyoxylsäureester so zudosiert, daß die Temperatur 90°C, vorzugsweise 80°C, nicht übersteigt und
B. das erhaltene Reaktionsgemisch mit Acetanhydrid behandelt.

Mit Hilfe des erfindungsgemäßen Verfahrens gelingt es, den begehrten 3-Formyl-crotonsäureethylester sowie die als Zwischenprodukte in der Wirkstoffchemie sehr interessanten 3-substituierten 3-Formyl-acrylsäureester auf recht vorteilhafte Weise in Ausbeuten von bis über 80 % herzustellen. Darüber hinaus werden neue interessante Zwischenprodukte zur Verfügung gestellt.

### Beispiel 1

(Vergleichsbeispiel in Anlehnung an DBP 1 008 729, Beispiel 1)

Zu einer bei 70°C siedenden Mischung aus 120 g (1 Mol) 2-Methoxy-2-hydroxy-essigsäuremethylester und 58 g (1 Mol) Propionaldehyd gab man 3,2 g (0,025 Mol) Di-n-butylamin. Sofort setzte eine exotherme Reaktion ein. Unter heftigem Sieden stieg die Temperatur auf 100°C an. Ein Teil der Reaktionsmasse siedete aus dem Kolben heraus. Die Temperatur ging langsam auf 90°C zurück und wurde hier 30 Minuten (min) gehalten. Fraktionierende Destillation der Reaktionsmischung ergab 38,9 g 3-Formyl-crotonsäuremethylester vom Siedepunkt (Sdp.) 56-68 °C/ 10 mbar, entsprechend einer Ausbeute von 30,4 % der Theorie. Das trans/cis-Verhältnis wurde gaschromatographisch zu 83/17 bestimmt.

### Beispiel 2

(Vergleichsbeispiel in Anlehnung an DBP 1 008 729, Beispiel 1)

Zu einer gerührten Mischung aus 88 g (1 Mol) Glyoxylsäuremethylester und 58 g (1 Mol) Propionaldehyd gab man bei 20 °C 3 g (0,025 Mol) Di-n-butylamin zu. Sofort setzt eine exotherme Reaktion ein. Die Temperatur der Reaktionsmasse stieg auf ca. 60 °C an. Die Reaktionsmischung siedete heftig. Nach ca. 1 min stieg die Temperatur erneut stark an und verharrte etwa 5 min bei ca. 130 °C. Danach fiel sie im Laufe von 20 min bis auf etwa 80 °C ab; nach dem Stehen über Nacht war sie auf Raumtemperatur abgesunken. Destillation der Reaktionsmischung ergab 75 g Produktfraktionen vom Sdp. 60 - 73 °C/30 mbar, welche insgesamt 68 g 3-Formylcrotonsäuremethylester, entsprechend einer Ausbeute von 53 % der Theorie, enthielten. Das trans/cis-Verhältnis wurde gaschromatographisch zu 84/16 bestimmt.

### Beispiel 3

(Vergleichsbeispiel in Anlehnung an DBP 1 008 729, Beispiel 1)

Zu einer gerührten Mischung aus 120 g (1 Mol) 2-Methoxy-2-hydroxy-essigsäuremethylester und 58 g (1 Mol) Propionaldehyd gab man bei 20 °C 3 g (0,025 Mol) Di-n-butylamin zu. Sofort setzte eine exotherme Reaktion ein. Die Temperatur der Reaktionsmasse stieg auf ca. 90 °C an. Die Reaktionsmischung siedete heftig. Nach ca. 10 min begann die Temperatur wieder abzusinken und erreichte nach ca. 1,5 h Raumtemperatur. Destillation der Reaktionsmischung ergab 66,4 g 3-Formyl-crotonsäuremethylester vom Sdp. 45-58 °C/ 8-9 mbar, entsprechend einer 52 g6igen Ausbeute. Das trans/cis-Verhältnis wurde gaschromatographisch zu 91/9 bestimmt.

### Beispiel 4

(Vergleichsbeispiel gegenüber DE 36 17 409))

Zu einer auf 50°C erwärmten Mischung aus 60 g (1 Mol) Essigsäure und 112,5 g (1 Mol) einer 40 gew.-%igen wäßrigen Dimethylaminlösung gab man unter Rühren und Kühlen mit Eiswasser innerhalb 10 min eine Mischung aus 88 g (1 Mol) Glyoxylsäuremethylester und 116 g (2 Mol) Propionaldehyd. Trotz Eiskühlung stieg die Temperatur auf ca. 70°C an.

Nach Beendetem Zutropfen wurde sofort mit Eiswasser abgekühlt, mit 200 ml Essigester versetzt, die organische Phase abgetrennt und die wäßrige Phase noch fünfmal mit Essigester extrahiert. Die vereinigten organischen Phasen wurden destilliert. Nach zweimaliger Destillation erhielt man 50 g 3-Formyl-crotonsäuremethylester vom Sdp. 54°C/1 mbar, entsprechend einer Ausbeute von 39 % der Theorie, bezogen auf den Glyoxylester. Das Produkt war rot gefärbt und zersetzte sich bei der Lagerung.

### Beispiel 5

In einem Rührkolben wurden 18 g (0,2 Mol) Sarkosin in 500 ml Cyclohexan suspendiert und beginnend bei 20 °C eine Mischung bestehend aus 1809 g (20,6 Mol) Glyoxylsäuremethylester und 1194 g (20,6 Mol) Propionaldehyd innerhalb von 90 min zugegeben. Die Temperatur stieg während des Zutropfens langsam bis zum Siedepunkt (75 °C) an. Nach beendeter Zugabe kreiste man zunächst geringe Mengen Wasser aus und destillierte dann bis zu einer Sumpftemperatur von 100 °C Cyclohexan ab. Anschließend wurden dem Rückstand 2220 g (21,8 Mol) Acetanhydrid zugesetzt und aus dem Reaktionsgemisch bis zu einer Sumpftemperatur von 150 °C Essigsäure abdestilliert. Fraktionierende Destillation des Sumpfprodukts ergab 1822 g (14,2 Mol) 3-Formyl-crotonsäuremethylester, entsprechend einer Ausbeute von 69 % der Theorie mit einem trans/cis-Isomerenverhältnis von 93/7.

### Beispiel 6

Zu einer vorgelegten Mischung aus 18 g (0,3 Mol) Essigsäure und 34 g (0,3 Mol) einer 40 gew.%igen wäßrigen Dimethylaminlösung wurde unter Kühlen und Rühren eine Mischung bestehend aus 264 g (3 Mol) Glyoxylsäuremethylester und 348 g (6 Mol) Propionaldehyd so zudosiert, daß die Reaktionstemperatur nicht über 70 °C anstieg. Nach beendeter Zugabe wurde abgekühlt und mehrmals mit Essigester extrahiert. Zur Abtrennung von Katalysatorresten wurden die Extrakte mit Mischbettionenaustauscher behandelt und anschließend destilliert. Man erhielt 236 g eines Destillats mit einem Siedebereich von 40 -75 °C/1 mbar, das lt. Gaschromatogramm zu 90 % aus 2-Hydroxy-3-methyl-butansäuremethylester bestand sowie 64 g Nachlauf mit einem Siedebereich von 75 - 100 °C, welcher 53 % 2-Hydroxy-3-methyl-butansäuremethylester enthielt. Die Ausbeute betrug somit insgesamt 246,3 g Hydroxyester, entsprechend 56 % der Theorie.

### Beispiel 7

Zu einer vorgelegten Mischung aus 1,85 g (0,025 Mol) Propionsäure und 3,22 g (0,025 Mol) Di-n-butylamin wurde eine Mischung aus 57 g (0,5 Mol) n-Heptanal und 44 g (0,5 Mol) Glyoxylsäuremethylester so zugetropft, daß die Temperatur 80 °C nicht überstieg. Nach beendetem Zutropfen wurde noch 30 min bei 80 °C gerührt, dann 102 g (1 Mol) Acetanhydrid zugegeben und unter Rückfluß zum Sieden erhitzt. Dabei destillierte langsam Essigsäure ab. Innerhalb von insgesamt 2 Stunden wurden 51 g Essigsäure abdestilliert. Durch fraktionierende Destillation des Sumpfprodukts erhielt man 59 g 3-Formyl-3-pentyl-acrylsäuremethylester mit einem Siedepunkt von 82 - 85°C/1 mbar, entsprechend einer Ausbeute von 64 % der Theorie. Das trans/cis-Isomerenverhältnis betrug 78/22.

### Beispiel 8

Zu einer Mischung aus 968 g (11 Mol) Glyoxylsäuremethylester, 352 g (11 Mol) Methanol und 9,6 g (0,11 Mol) Sarkosin gab man bei 50°C innerhalb 45 min. 688 g (11,9 Mol) Propionaldehyd. Nach beendeter Zugabe wurde 1 h bei 80°C nachgerührt, dann 2805 g (27,5 Mol) Acetanhydrid zugesetzt. Bei Normaldruck wurde über eine Kolonne bis zur Kopftemperatur von 105°C Methylacetat/Essigsäure abdestilliert. Anschließend wurde bei Normaldruck bis 125°C ein Gemisch aus Methylacetat, Essigsäure, Acetanhydrid und 3-Formyl-crotonsäuremethylester entnommen, dann zunächst bei 250 mbar bis 105°C, anschließend bei 30 mbar bis 90°C Kopftemperatur weiterdestilliert. Die vereinigten Destillate wurden rektifiziert. Man erhielt 1184 g (9,3 Mol) 3-Formylcrotonsäuremethylester, entsprechend einer Ausbeute von 84 % der Theorie.

### Beispiel 9

Zu einer vorgelegten Mischung aus 264 g (3 Mol) Glyoxylsäuremethylester, 96 g Methanol und 2,7 g (0,03 Mol) Sarkosin gab man bei 80°C innerhalb von 1 h 174 g (3 Mol) Propionaldehyd. Nach beendeter Zugabe wurde das Reaktionsgemisch noch 1 h bei 80°C gerührt. Anschließend wird das Reaktionsgemisch bei einem Druck von 1 mbar in einem Dünnschichtverdampfer destilliert. Man erhielt 417 g Destillat, das 50,8 96 2-Hydroxy-3-formylbutansäuremethylester und 16,4 96 Formylcrotonsäuremethylester enthielt.

## Patentansprüche

1. 3-Substituierte 2-Hydroxy-3-formyl-propionsäureester der allgemeinen Formel I 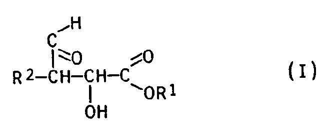 in der
R¹ für einen Alkylrest mit 1 bis 4 C-Atomen steht und
R² für einen geradkettigen oder verzweigten Alkylrest mit 1 bis 10 C-Atomen steht.

2. 3-Formyl-2-hydroxy-butansäurealkylester der allgemeinen Formel Ia 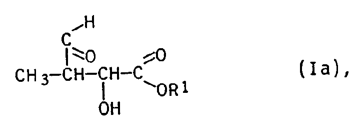 in der
R¹ für einen Alkylrest mit 1 bis 4 C-Atomen steht.

3. Verfahren zur Herstellung von 3-substituierten 2-Hydroxy-3-formylpropionsäureestern der allgemeinen Formel I gemäß Anspruch 1 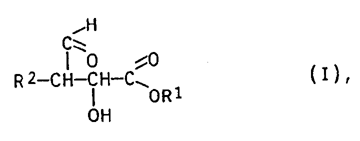 in der
R¹ und R² die in Anspruch 1 angegebene Bedeutung haben, dadurch gekennzeichnet, daß man
ein Alkanal der allgemeinen Formel II 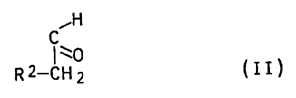 und einen Glyoxylsäurealkylester der allgemeinen Formel III 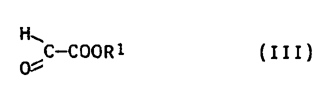 in denen
R¹ und R² die oben angegebene Bedeutung haben, gleichzeitig zu einem Katalysatorsystem bestehend aus einem Salz eines sekundären Amins mit einer Carbonsäure oder einem etwa äquimolekularen Gemisch aus einem sekundären Amin und einer Carbonsäure oder einer Verbindung, die eine sekundäre Aminogruppe und eine Carboxylgruppe im gleichen Molekül enthält, so zudosiert, daß die Temperatur 90°C nicht übersteigt, oder aber das Alkanal der Formel II langsam zu einem Gemisch aus dem oben beschriebenen Katalysatorsystem und dem Glyoxylsäureester so zudosiert, daß die Temperatur 90°C nicht übersteigt, wobei das Katalysatorsystem in Mengen von 0,01 bis 10 Mol-%, bezogen auf den Glyoxylsäureester verwendet wird.

4. Verfahren gemäß Anspruch 3, dadurch gekennzeichnet, daß man als Alkanal der Formel II Propionaldehyd verwendet.

5. Verfahren gemäß Anspruch 3, dadurch gekennzeichnet, daß man die gemäß diesem Verfahren erhaltenen 2-Hydroxy-3-formyl-propionsäureester der allgemeinen Formel I zur Herstellung von 3-substituierten 3-Formyl-acrylsäureestern der allgemeinen Formel IV 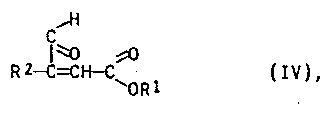 in der
R¹ und R² die in Anspruch 1 angegebene Bedeutung haben, mit einem wasserabspaltenden Mittel behandelt.

6. Verfahren gemäß Anspruch 3, dadurch gekennzeichnet, daß man das gemäß diesem Verfahren erhaltene, 2-Hydroxy-3-formyl-propionsäureester der allgemeinen Formel I enthaltende, Reaktionsgemisch zur Herstellung von 3-substituierten 3-Formyl-acrylsäureestern der allgemeinen Formel IV 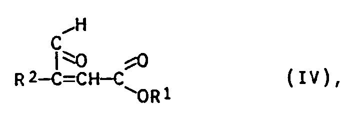 in der R¹ und R² die in Anspruch 1 angegebene Bedeutung haben, mit Acetanhydrid behandelt.

7. Verwendung der 3-substituierten 2-Hydroxy-3-formyl-propionsäureester der allgemeinen Formel I gemäß Anspruch 1 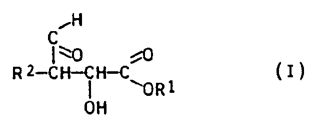 in der
R¹ und R² die in Anspruch 1 angegebene Bedeutung haben, zur Herstellung von 3-Formyl-acrylsäureestern der allgemeinen Formel IV 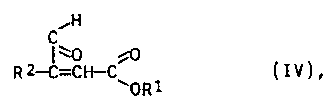 in der
R¹ und R² die in Anspruch 1 angegebene Bedeutung haben.

8. Verfahren zur Herstellung von 3-Formyl-crotonsäure-alkylestern der allgemeinen Formel IVa 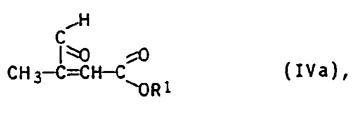 in der
R¹ für einen Alkylrest mit 1 bis 4 C-Atomen steht, dadurch gekennzeichnet, daß man
A. Propionaldehyd und eien Glyoxylsäurealkylester der allgemeinen Formel III 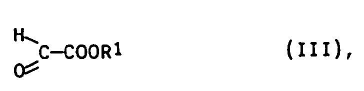 in der R¹ die oben angegebene Bedeutung hat,
entweder gleichzeitig zu einem Katalysatorsystem bestehend aus einem Salz eines sekundären Amins mit einer Carbonsäure oder einem etwa äquimolaren Gemisch aus einem sekundären Amin und einer Carbonsäure oder einer Verbindung, die eine sekundäre Aminogruppe und eine Carboxylgruppe im gleichen Molekül, enthält, so zudosiert, daß die Temperatur 90°C nicht übersteigt, oder aber das Alkanal der Formel II zu einem Gemisch aus dem oben beschriebenen Katalysatorsystem und dem Glyoxylsäureester der Formel III so zudosiert, daß die Temperatur 90°C nicht übersteigt und
B. das erhaltene Reaktionsgemisch mit Acetanhydrid behandelt.

## Claims

1. A 3-substituted 2-hydroxy-3-formylpropionic ester of the formula I 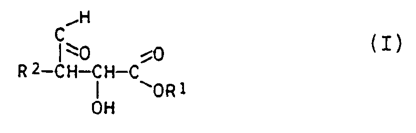 where
R¹ is alkyl of 1 to 4 carbon atoms, and
R² is straight-chain or branched alkyl of 1 to 10 carbon atoms.

2. An alkyl 3-formyl-2-hydroxybutanoate of the formula Ia 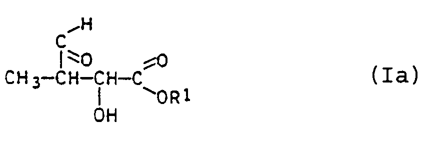 where
R¹ is alkyl of 1 to 4 carbon atoms.

3. A process for preparing a 3-substituted 2-hydroxy-3-formylpropionic ester of the formula I as claimed in claim 1 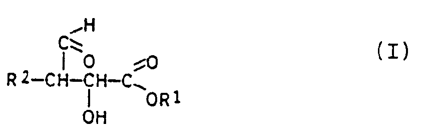 where
R¹ and R² have the meanings stated in claim 1, which comprises
an alkanal of the formula II 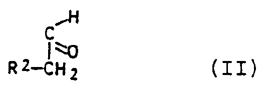 and an alkyl glyoxylate of the formula III 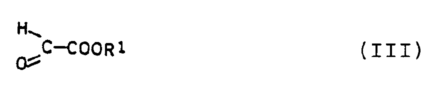 where R¹ and R² have the abovementioned meanings, being added simultaneously to a catalyst system composed of a salt of a secondary amine with a carboxylic acid or an approximately equimolar mixture of a secondary amine and a carboxylic acid, or a compound which contains a secondary amino group and a carboxyl group, in such a way that the temperature does not exceed 90°C, or else the alkanal of the formula II being added slowly to a mixture of the catalyst system described above and the glyoxylic ester in such a way that the temperature does not exceed 90°C, where the catalyst system is used in an amount of from 0.01 to 10 mol %, based on the glyoxylic ester.

4. A process as claimed in claim 3, wherein propionaldehyde is used as alkanal of the formula II.

5. A process for preparing a 3-substituted 3-formylacrylic ester of the formula IV 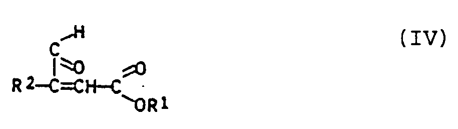 where
R¹ and R² have the meanings stated in claim 1, which comprises a 2-hydroxy-3-formylpropionic ester of the formula I obtained by the process claimed in claim 3 being treated with a dehydrating agent.

6. A process for preparing a 3-substituted 3-formylacrylic ester of the formula IV 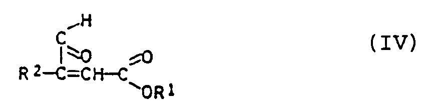 where R¹ and R² have the meanings stated in claim 1, which comprises the reaction mixture which is obtained by the process claimed in claim 3 and contains a 2-hydroxy-3-formylpropionic ester of the formula I being treated with acetic anhydride.

7. The use of a 3-substituted 2-hydroxy-3-formylpropionic ester of the formula I as claimed in claim 1 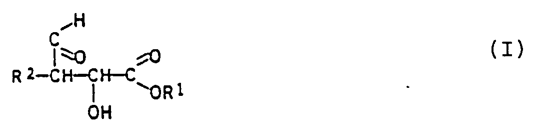 where
R¹ and R² have the meanings stated in claim 1, for preparing a 3-formylacrylic ester of the formula IV 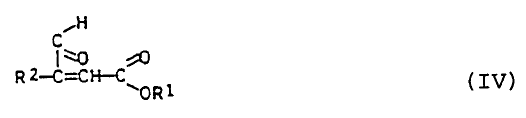 where R¹ and R² have the meanings stated in claim 1.

8. A process for preparing an alkyl 3-formylcrotonate of the formula IVa 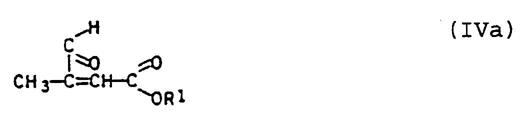 where
R¹ is alkyl of 1 to 4 carbon atoms, which comprises
A. either adding propionaldehyde and an alkyl glyoxylate of the formula III 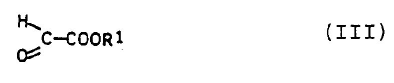 where R¹ has the abovementioned meaning,
simultaneously to a catalyst system composed of a salt of a secondary amine with a carboxylic acid or an approximately equimolar mixture of a secondary amine and a carboxylic acid, or a compound which contains a secondary amino group and a carboxyl group, in such a way that the temperature does not exceed 90°C, or adding the alkanal of the formula II to a mixture of the catalyst system described above and the glyoxylic ester of the formula III in such a way that the temperature does not exceed 90°C, and
B. treating the resulting reaction mixture with acetic anhydride.

## Revendications

1. Esters substitués en position 3 de l'acide 2-hydroxy-3-formylpropionique répondant à la formule générale I 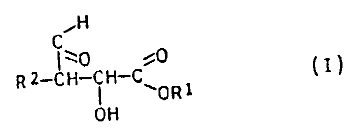 dans laquelle
R¹ est un radical alkyle ayant de 1 à 4 atomes de carbone, et
R² est un radical alkyle à chaîne droite ou ramifiée ayant de 1 à 10 atomes de carbone.

2. Esters alkyliques de l'acide 3-formyl-2-hydroxy-butanoïque répondant à la formule générale Ia 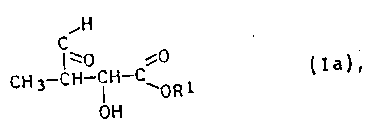 dans laquelle R¹ est un radical alkyle ayant de 1 à 4 atomes de carbone.

3. Procéde pour préparer des esters substitués en position 3 de l'acide 2-hydroxy-3-formylpropionique, répondant à la formule générale I, selon la revendication 1, 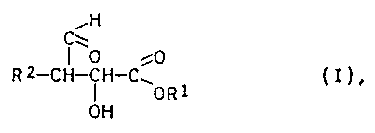 dans laquelle
R¹ et R² ont les significations données dans la revendication 1, caractérisé en ce qu'on ajoute simultanément un alcanal formule générale II 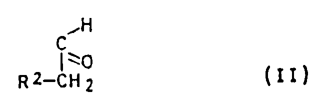 et un ester alkylique de l'acide glyoxylique répondant à la formule générale III 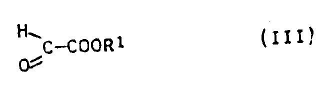 formules dans lesquelles R¹ et R² ont les significations données ci-dessus, à un système catalytique constitué d'un sel d'une amine secondaire et d'un acide carboxylique ou d'un mélange approximativement équimolaire d'une amine secondaire et d'un acide carboxylique, ou d'un composé contenant, dans une seule et même molécule, un groupe amino secondaire et un groupe carboxyle, de façon que la température ne dépasse pas 90°C, ou encore en ce qu'on ajoute lentement l'alcanal répondant à la formule II à un mélange du système catalytique décrit ci-dessus et de l'ester de l'acide glyoxylique, de façon que la température ne dépasse pas 90°C, le système catalytique étant utilisé en des quantités comprises entre 0,01 et 10 % en moles par rapport à l'ester de l'acide glyoxylique.

4. Procédé selon la revendication 3, caractérisé en ce qu'on utilise comme alcanal répondant à la formule II le propionaldéhyde.

5. Procédé selon la revendication 3, caractérisé en ce qu'on traite, avec un agent éliminant l'eau, les esters de l'acide 2-hydroxy-3-formylpropionique obtenus par ce procédé, répondant à la formule générale I, pour préparer des esters substitués en position 3 de l'acide 3-formylacrylique répondant à la formule générale IV 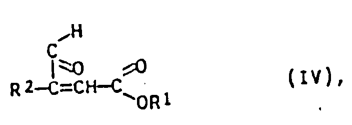 dans laquelle R¹ et R² ont les significations données dans la revendication 1.

6. Procédé selon la revendication 3, caractérisé en ce qu'on traite, par de l'anhydride acétique le mélange réactionnel obtenu par ce procédé et contenant un ester de l'acide 2-hydroxy-3-formylpropionique répondant à la formule générale I, pour préparer des esters substitués en position 3 de l'acide 3-formylacrylique répondant à la formule générale IV 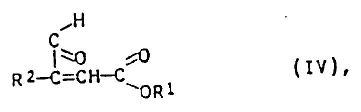 dans laquelle R¹ et R² ont les significations données dans la revendication 1.

7. Utilisation des esters substitués en position 3 de l'acide 2-hydroxy-3-formylpropionique répondant à la formule générale I selon la revendication 1 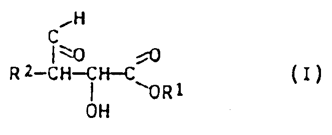 dans laquelle R¹ et R² ont les significations données dans la revendication 1, pour préparer des esters de l'acide 3-formylacrylique répondant à la formule générale IV 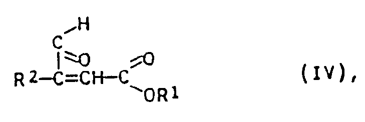 dans laquelle R¹ et R² ont les significations données dans la revendication 1.

8. Procédé pour préparer des esters alkyliques de l'acide 3-formylcrotonique répondant à la formule générale IVa 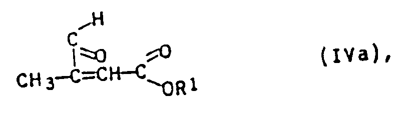 dans laquelle R¹ est un radical alkyle ayant de 1 à 4 atomes de carbone, caractérisé en ce que
A. on ajoute simultanément du propionaldéhyde et un ester alkylique de l'acide glyoxylique répondant à la formule générale III 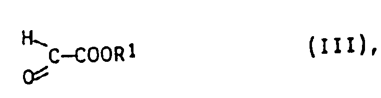 dans laquelle R¹ a la signification donnée ci-dessus, à un système catalytique constitué d'un sel d'une amine secondaire et d'un acide carboxylique ou d'un mélange approximativement équimolaire d'une amine secondaire et d'un acide carboxylique ou d'un composé contenant dans une seule et même molécule, un groupe amino secondaire et un groupe carboxyle, de façon que la température ne dépasse pas 90°C, ou encore on ajoute l'alcanal répondant à la formule II, à un mélange du système catalytique décrit ci-dessus et de l'ester de l'acide glyoxylique répondant à la formule III, de façon que la température ne dépasse pas 90°C, et
B. on traite à l'anhydride acétique le mélange réactionnel obtenu.
